# EUROPEAN PATENT SPECIFICATION

(11) **EP 0 604 708 B1**
(45) Date of publication and mention of the grant of the patent: **07.07.1999**
(21) Application number: 93113598.2
(22) Date of filing: 07.03.1989
(51) Int. Cl.: C12P 5/02, C02F 11/04

(54) **Fermentation method for the fast production of methane**
Fermentationsverfahren zur schnellen Methanproduktion
Méthode de fermentation pour la production rapide de méthane

(30) Priority: 07.03.1988 JP 5329488; 07.03.1988 JP 5329588
(43) Date of publication of application: 06.07.1994
(62) Divisional of application: 89103989.3
(73) Proprietor: Research Development Corporation of Japan, Chiyoda-ku Tokyo (JP); MITSUBISHI ELECTRIC CORPORATION, Tokyo 100 (JP); RIKAGAKU KENKYUSHO, Wako-shi Saitama-ken (JP)
(72) Inventor: Nakatsugawa, Naoki, Wako-shi, Saitama-ken (JP); Horikoshi, Koki, Tokyo (JP)
(74) Representative: VOSSIUS & PARTNER

(56) References cited:
- DISSERTATION ABSTRACTS INT.B,Vol. 32, No.9 , 1972, page 5238-B G.H.TOENNIESSEN, "Studies on acetate and methanol fermenting methanogenic enrich."
- PATENT ABSTRACTS OF JAPAN vol. 4, no. 19 (C-73)16 February 1980 & JP-A-54 154 597 (MITSUBISHI JUKOGYO K.K.) 12 May 1979

## Description

The present invention relates to a methane fermentation method in which methanogenic bacteria are used and more specifically to a method for carrying out methane fermentation in a reactor for methane fermentation at a high speed within a short period of time in which the concentration of methanogenic bacteria is increased, and the best use of the properties of the methanogenic bacteria is made.

Heretofore, there have been known the following 5 bacterial species as methanogenic bacteria belonging to the genus Methanosarcina: Methanosarcina barkeri (see Microbiological Reviews, 1979, Vol. 47, pp. 260-296 and FEMS Microbiology Letters, 1983, Vol. 16, pp. 217-223); Methanosarcina acetivorans (see Applied and Environmental Microbiology, 1984, Vol. 47, No.5, pp. 971-978); Methanosarcina vacuolata (see Microbiology, U.S.S.R., 1979, Vol. 48, pp. 279-285); Methanosarcina thermophila (see International Journal of Systematic Bacteriology, 1985, Vol. 35, No.4, pp. 522-523); and Methanosarcina mazei (see Current Microbiology, 1980, Vol. 3, pp. 321-326). These species can be used for the method of the invention.

Besides, methane fermentation methods have been utilized widely for treating waste water and solid waste from the viewpoint of effective use of biomass, biogas production or the like.

Nevertheless, the conventionally known methanogenic bacteria used in methane fermentation methods are strictly anaerobic bacteria and show an extremely slow growth rate. For this reason, the use of a large-scale reactor for methane fermentation is required and likewise it takes a long period of time to obtain in the reactor the concentration of the methanogenic bacteria which is required for maintaining a stable steady state of methane fermentation.

There has been proposed a method which comprises the step of controlling conditions in the reactor such as pH and temperature for the purpose of improving the efficiency of the foregoing methane fermentation. However, such a control of only the environmental conditions enables such a method to be speeded up only slightly and permits an improvement of efficiency only to a limited level.

Furthermore, a method of methane fermentation utilizing organic waste is disclosed, characterized by using methane bacteria such as methanosulcina, methanococcus or methanobacterium and adding lower aliphatic acid or alcohol such as acetic acid, butyric acid, methyl alcohol or ethyl alcohol to the organic waste (see Patent Abstracts of Japan, 1980, Vol. 4, No. 19 (C-73)).

In particular, in the methane fermentation methods for treating solid waste and/or waste water, the main substrates are acetic acid and H₂/CO₂ (among the substrates capable of being assimlated by methanogens, acetic acid and H₂/CO₂ are exclusively present in such waste). However, the assimilation rate of acetic acid by methanogenic bacteria is very low and this serves as a rate-determining step. Thus, it takes a long period of time to start the methane fermentation reactor and the volume of the reactor must be enlarged. Morever, the reactor is started according to the adaptation-cultivation method using substrates inherent to the bacteria used.

Accordingly, an object of the present invention is to provide a methane fermentation method which can provide methane at a high speed and high efficiency and which makes it possible to increase the bacterial concentration in a reactor within a short period of time, utilizing the bacteriological properties of methanogenic bacteria.

According to the present invention, there is provided a methane fermentation method which comprises culturing at least one methanogenic bacterium in a culture medium, wherein the methanogenic bacterium is capable of assimilating one or more first substrates such as acetic acid, H₂/CO₂, or a mixture thereof at a low rate and a second substrate at a high rate, the culture medium containing one or more said first substrates and the culture being carried out in the additional presence of methylamine as the second substrate.

The present invention will hereunder be explained in more detail with reference to the accompanying drawing, wherein,

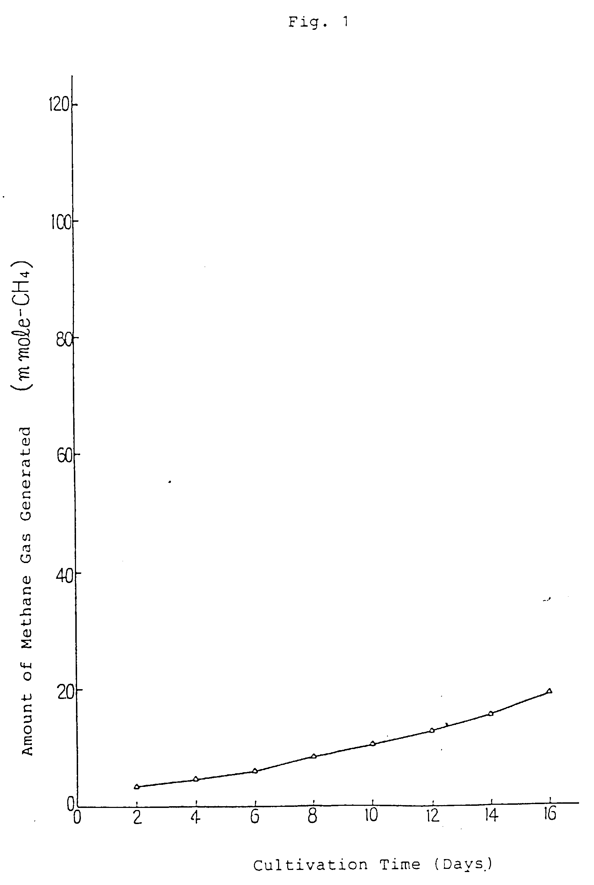

Fig. 1 is a diagram illustrating the change in the amount of methane gas generated with time.

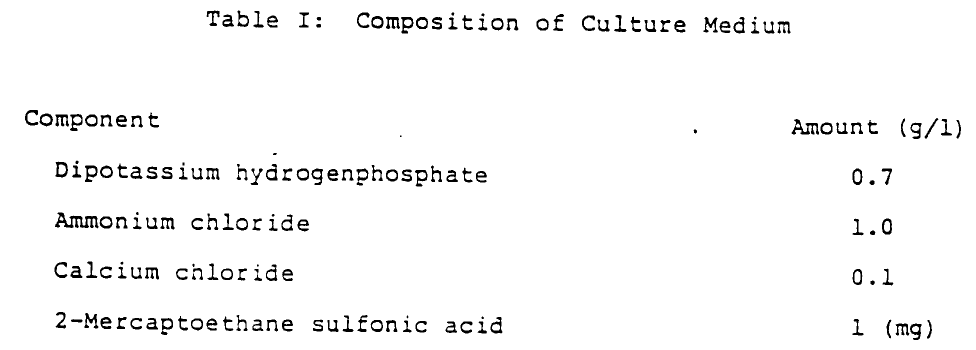

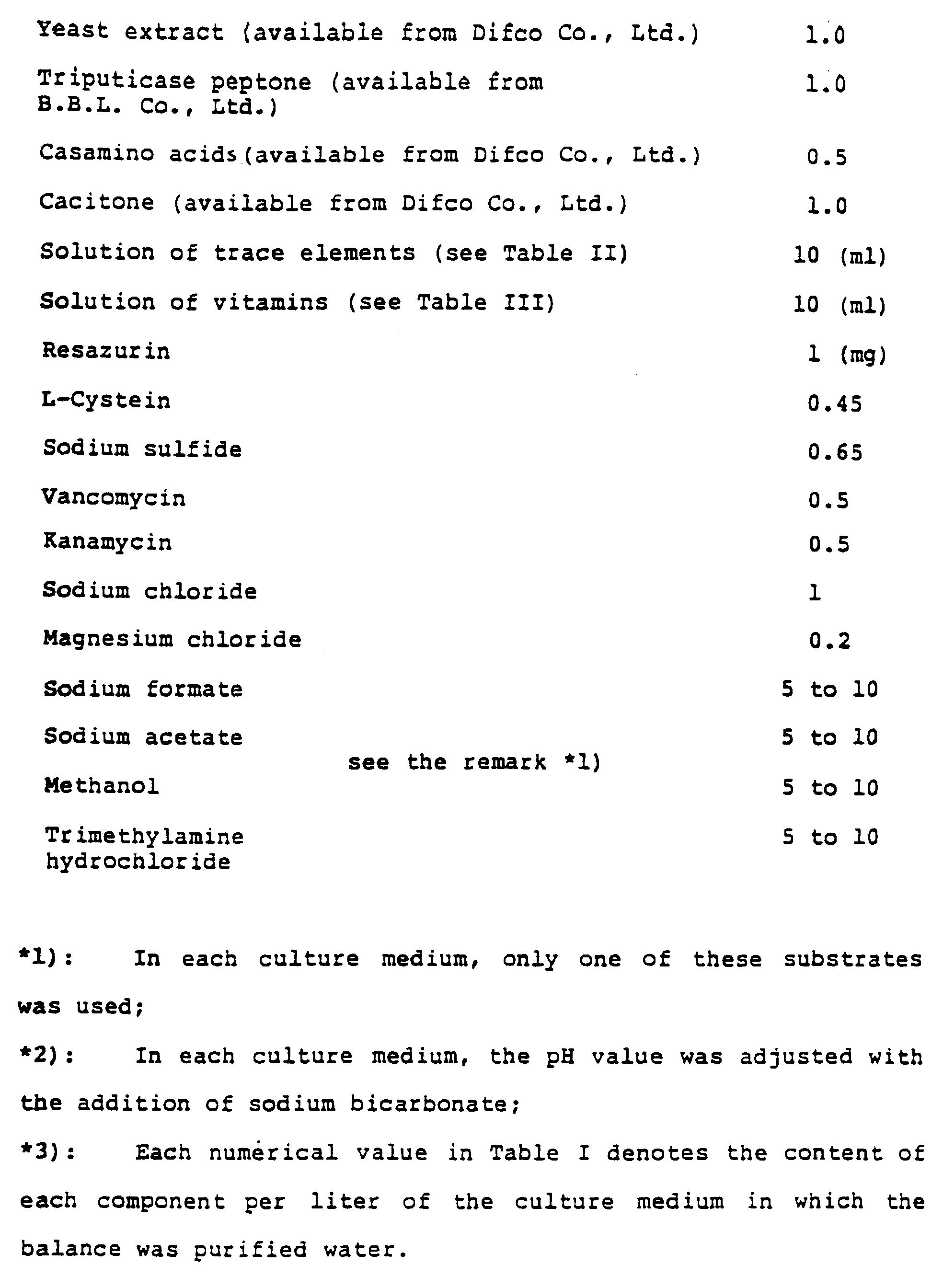

**Table II:**

| Composition of the Solution of Trace Element | |
|---|---|
| Component | Amount (g/l) |
| Ferrous sulfate | 0.3 |
| Cobalt chloride | 0.2 |
| Zinc chloride | 0.1 |
| Boric acid | 0.05 |
| Sodium molybdate | 0.2 |
| Manganese chloride | 0.1 |
| Copper sulfate | 0.01 |
| Aluminum potassium sulfate | 0.005 |
| Nitrilotriacetic acid | 2.5 |
| Nickel chloride | 0.5 |
| Sodium selenite | 0.1 |
| Sodium tungstate | 1.0 |
| Cadmium chloride | 0.05 |
| *): Each numerical value in Table II denotes the content of each component per liter of the solution of trace elements in which the balance was purified water. | |

**Table III:**

| Composition of the Solution of Vitamin | |
|---|---|
| Component | Amount (mg/l) |
| Biotin | 2 |
| Folic acid | 2 |
| Pyridoxine | 10 |
| Riboflavin | 5 |
| Thiamine | 5 |
| Pantothenic acid | 5 |
| Cyanocobalamin | 0.1 |
| p-Aminobenzoic acid | 5 |
| Alpha-lipoic acid | 5 |
| *): Each numerical value in Table III denotes the content of each component per liter of the solution of vitamins in which the balance was purified water. | |

The methane fermentation method of the present invention will hereunder be explained in more detail.

In the method of the present invention, there are employed methanogenic bacteria which are capable of utilizing at least two substrates and which utilize these substrates at different assimilation rates.

Examples of such methanogenic bacteria include those belonging to the genus Methanosarcina. Specific examples of such: methanogenic bacteria are listed in the following Table IV.

The method of the present invention produces methane by culturing the aforementioned methanogenic bacteria in the presence of substrates as principal subject of fermentation which are assimilated by the bacteria at a low assimilation rate such as acetic acid, H₂/CO₂, or a mixture thereof and carrying out the culture or the fermentation in the presence of substrates which are assimilated at a low assimilation rate and of the substrate methylamine which is assimilated at a rate faster than that of the former.

For instance, when Methanosarcina acetivorans C2A is cultured at a temperature of 35 to 40°C, the generation time thereof is 24.1 hours for acetic acid as a substrate; 5.2 hours for methanol; and 6.7 to 7.3 hours for methylamine.

If acetic acid is used as the substrate which is assimilated at a low rate, methylamine is used as the substrate assimilated at a high rate.

The method of the present invention can likewise be carried out in the same manner as above, when Methanosarcina alkaliphilum, (whose generation time at 37°C is 19 hours for H₂/CO₂; 17 hours for acetic acid; 4 hours for methanol; and 6 hours for methylamine) is used as the methanogenic bacterium. For instance, when H₂/CO₂, acetic acid or a mixture thereof is used as the substrate which is assimilated at a low rate, methylamine is used as the substrate which is assimilated at a high assimilation rate.

The method of the present invention can be performed in the presence of one or more of the substrates such as acetic acid, H₂/CO₂ or a mixture thereof which are assimilated at a low assimilation rate and of the substrate methylamine which is assimilated at a high assimilation rate, simultaneously.

The time for introducing, into a reaction system, the substrate assimilated at a high rate and the amount thereof may vary depending on factors such as the scale of the fermentation bath. For instance, the starting time of the reaction can extremely be reduced by adding it at the beginning of the fermentation.

Conditions of fermentation may appropriately be varied depending on the kinds of the methanogenic bacteria and the fermentation may be carried out according to conventional methods.

The method of the present invention can be applied to not only the methane fermentation in treating solid waste and waste water but also to the methane fermentation in all the other fields.

The methane fermentation method of the present invention makes it possible to increase the bacterial concentration of the methanogenic bacterium in a reactor within a very short time. Therefore, the size of the reactor can be made compact substantially, by the use of such methanogenic bacteria. In addition, the time required for establishing a desired bacterial cell number in the reactor can also be reduced substantially and thus a stable steady state of methane fermentation can be maintained.

**Table IV**

| Name of Bacteria (accession No.) | Substrate Assimilation Properties | | | | |
|---|---|---|---|---|---|
| | H₂/CO₂ | HCOOH | MeCOOH | MeOH | MeNH₂ |
| (i) Known methanogens belonging to genus Methanosarcina | | | | | |
| Methanosarcina barkeri MS (DSM 800^{T}) *1) | + | - | + | + | + |
| Methanosarcina barkeri 227 (DSM 1538) *1) | + | - | + | + | + |
| Methanosarcina barkeri UBS (DSM 1311) *1) | + | - | + | + | + |
| Methanosarcina barkeri DM *1) | + | - | + | + | + |
| Methanosarcina barkeri FRI (DSM 2256) *2) | + | - | + | + | + |
| Methanosarcina acetivorans C2A (DSM 2834^{T}) *3) | - | - | + | + | + |
| Methanosarcina vacuolata Z (DSM 1232) *4) | + | - | + | + | + |
| Methanosarcina thermophila TMI (DSM 1825^{T}) *5) | - | - | + | + | + |
| Methanosarcina mazei (DSM 2053) *6) | - | - | + | + | + |
| (ii) Known alkalophilic methanogens | | | | | |
| Methanosarcina alkaliphilum WeN₄ *7) | + | - | - | - | - |
| Methanosarcina thermoalkaliphilum Ac60 *8) | + | - | - | - | - |

| Name of Bacteria (accession No.) | Morphology | Gramstain | Optimum Temp °C | Optimum pH | Generation Time(h) |
|---|---|---|---|---|---|
| (i) Known methanogens belonging to genus Methanosarcina | | | | | |
| Methanosarcina barkeriMS (DSM 800^{T}) | sarcina | + | 40 | 7.0 | 12 |
| Methanosarcina barkeri 227 (DSM 1538) | ditto | + | 35 | 7.0 | 24 |
| Methanosarcina barkeri UBS (DSM 1311) | ditto | + | 30 | 7.4 | 30 |
| Methanosarcina barkeri DM | ditto | + | 37 | 7.0 | 28 |
| Methanosarcina barkeri FRI (DSM 2256) | ditto | + | 35 | --- | 33 |
| Methanosarcina acetivorans C2A (DSM 2834^{T}) | ditto | - | 35-40 | 7.0 | 5.2 |
| Methanosarcina vacuolata Z (DSM 1232) | ditto | + | 40 | 6.5-7.0 | --- |
| Methanosarcina thermophila TMI (DSM 1825^{T}) | ditto | + | 50 | 7.5 | 5 |
| Methanosarcina mazei (DSM 2053) | ditto | ± | 30-40 | 6.0-7.0 | 7.7 |
| (ii) Known alkalophilic methanogens | | | | | |
| Methanosarcina alkaliphilum WeN₄ | rods | - | 37 | 8.1-9.1 | --- |
| Methanosarcina thermoalkaliphilum Ac60 | ditto | --- | 58-62 | 7.5-8.5 | --- |

| | | | | | |
|---|---|---|---|---|---|
| *1): Microbiological Reviews, 1979, Vol. 47, pp. 270-296; | | | | | |
| *2): FEMS Microbiology Letters, 1983, Vol. 16, pp. 217-223; | | | | | |
| *3): Applied and Environmental Microbiology, 1984, Vol. 47, No.5, pp. 971-978; | | | | | |
| *4): Microbiology (U.S.S.R.), 1979, Vol. 48, pp. 279-285; | | | | | |
| *5) : International Journal of Systematic Bacteriology, 1985, Vol. 35, No.4, pp. 533-523; | | | | | |
| *6): Current Microbiology, 1980, Vol. 3, pp. 321-326; | | | | | |
| *7) : International Journal of Systematic Bacteriology, 1986, Vol. 36, No.3, pp. 380-382; | | | | | |
| *8): Archives of Microbiology, 1985, Vol. 142, pp. 211-217. | | | | | |

### Example 1

Methanosarcina barkeri UBS (DSM 1311) was cultured, for 16 days, in one liter of the media (pH 7.4; Temp. = 37°C) having the compositions listed in Table I and containing 0.8% sodium acetate as the substrate respectively. The results obtained are plotted on Fig. 1.

## Claims

1. A methane fermentation method which comprises culturing at least one methanogenic bacterium in a culture medium, wherein the methanogenic bacterium is capable of assimilating one or more first substrates such as acetic acid, H₂/CO₂, or a mixture thereof at a low rate and a second substrate at a high rate, the culture medium containing one or more said first substrates and the culture being carried out in the additional presence of methylamine as the second substrate.

2. The methane fermentation method according to claim 1 wherein the methanogenic bacterium (bacteria) belongs (belong) to the genus Methanosarcina.

3. The methane fermentation method according to claim 1 or 2 wherein said part of the substrate which is assimilated at a low assimilation rate is acetic acid.

## Patentansprüche

1. Verfahren zur Methanfermentation, das die Züchtung von mindestens einem methanogenen Bakterium in einem Kulturmedium umfaßt, wobei das methanogene Bakterium fähig ist zur Umsetzung von einem oder mehreren ersten Substraten, wie Essigsäure, H₂/CO₂ oder einem Gemisch hiervon, bei niedriger Umsatzrate und einem zweiten Substrat bei hoher Umsatzrate, wobei das Kulturmedium eines oder mehrere der ersten Substrate beinhaltet, und die Kultur bei zusätzlicher Anwesenheit von Methylamin als zweitem Substrat durchgeführt wird.

2. Verfahren zur Methanfermentation nach Anspruch 1, wobei das (die) methanogene(n) Bakterium (Bakterien) zur Art Methanosarcina gehört (gehören).

3. Verfahren zur Methanfermentation nach Anspruch 1 oder 2, wobei der Teil des Substrates, der bei niedriger Umsetzungsrate umgesetzt wird, Essigsäure ist.

## Revendications

1. Procédé de fermentation de méthane, lequel comprend la culture d'au moins une bactérie méthanogène dans un milieu de culture, où la bactérie méthanogène est capable d'assimiler un ou plusieurs premiers substrats tels qu'acide acétique, H₂/CO₂ ou leur mélange à une vitesse faible et un second substat à une vitesse élevée, le milieu de culture contenant un ou plusieurs dits premiers substrats et la culture étant effectuée en présence additionnelle de méthylamine comme second substrat.

2. Procédé de fermentation de méthane selon la revendication 1, où la ou les bactéries méthanogènes appartiennent au genre Methanosarcina.

3. Procédé de fermentation de méthane selon la revendication 1 ou 2, où ladite partie du substrat qui est assimilée à une vitesse d'assimilation faible est de l'acide acétique.
